# EUROPEAN PATENT SPECIFICATION

(11) **EP 4 182 934 B1**
(45) Date of publication and mention of the grant of the patent: **20.05.2026**
(21) Application number: 21751746.5
(22) Date of filing: 13.07.2021
(51) Int. Cl.: G16H 20/00

(54) **A SECURE, AUTOMATED, SYSTEM AND COMPUTER IMPLEMENTED PROCESS FOR MONITORING CARE PROVIDED TO A CONSUMER ACCORDING TO A PHARMACOLOGICAL OR NUTRITIONAL REGIMEN OF THE CONSUMER**
SICHERES, AUTOMATISIERTES SYSTEM UND COMPUTERIMPLEMENTIERTES VERFAHREN ZUR ÜBERWACHUNG DER VERSORGUNG EINES VERBRAUCHERS NACH EINEM PHARMAKOLOGISCHEN ODER ERNÄHRUNGSPLAN DES VERBRAUCHERS
SYSTÈME SÉCURISÉ, AUTOMATISÉ ET PROCÉDÉ MIS EN OEUVRE PAR ORDINATEUR POUR SURVEILLER DES SOINS DONNÉS À UN CONSOMMATEUR EN FONCTION D'UN RÉGIME PHARMACOLOGIQUE OU NUTRITIONNEL DU CONSOMMATEUR

(30) Priority: 14.07.2020 CH 8692020; 14.07.2020 US 202016928072
(43) Date of publication of application: 24.05.2023
(73) Proprietor: Blunergy SA, 1015 Lausanne VD (CH)
(72) Inventor: MOSSIER, Evan, 1004 Lausanne VD (CH); KÃUPER, Peter, 1003 Lausanne VD (CH); BAUDE, Adrien, 1006 Lausanne VD (CH); KELLER, Matteo, 6517 Arbedo (CH); ROSSI, Nathanaël, 1110 Morges VD (CH); SOLIGNAC, Dominique, 1305 Penthalaz (CH)
(74) Representative: Schneiter, Sorin
(86) International application number: PCT/EP2021/069533
(87) International publication number: WO 2022/013259

(56) References cited:
- WO-A1-2020/035282
- US-A1- 2020 152 312
- MCLLWRAITH DOUGLAS GAVIN: "Ambient and Wearable Sensor Fusion for Privacy Respectful Pervasive Monitoring Environments", 1 December 2010 (2010-12-01), XP055857920, Retrieved from the Internet <URL:https://spiral.imperial.ac.uk/bitstream/10044/1/74698/1/Mcllwraith-DG-PhD-Thesis.pdf> [retrieved on 20211104]

## Description

### TECHNICAL DOMAIN

The present disclosure relates generally to the optimisation of a production process, and more particularly to its application in a hospitality environment. Embodiments described herein may find particular use where optimised control of the preparation of meals for patients is sought, especially where patients' dietary and nutritional requirements and general well-being are to be considered.

More particularly, embodiments disclosed herein may find use in a patient care facility where computer vision technology is deployed in a system for monitoring one or more aspects related to the patient's wellbeing or for monitoring a regimen followed by the patient while guaranteeing the patient's privacy or anonymity, especially where such computer vision technology needs to be maintained or improved over time, after deployment, by personnel to whom access to information related to the patient's wellbeing or regimen is to be prevented as part of a method for ensuring the patient's privacy or anonymity.

Similarly, an embodiment may find use in a patient care facility where computer vision technology is deployed in a system for verifying that a meal prepared for a particular patient is correctly prepared according to pre-defined requirements of the intended patient and/or correctly delivered to the intended patent, while guaranteeing the patient's privacy or anonymity, especially where such computer vision technology needs to be maintained or improved over time, after deployment, by personnel to whom access to information related to the patient's wellbeing or regimen is to be prevented as part of a method for ensuring the patient's privacy or anonymity.

As well as in care environments, embodiments described herein may find use in other environments such as schools, prisons, training camps, holiday camps or any other institution or environment in which a nutritional or pharmacological regimen of consumers within that environment is to be monitored or otherwise controlled.

WO 2020/35282 discloses a system for optimizing meal preparation in a patient-care environment, comprising an inspection unit for taking a measurement of a serving before and after consumption. US 2020/152312 discloses a nutritional monitoring a management system comprising a wearable sensor suitable to detect eating, so as to provide passive feedback concerning consumed food types and quantities.

### TECHNICAL BACKGROUND

Plate waste is a well-known phenomenon in the care domain. Techniques are known for measuring food waste through weighing and/or through manual visual estimation of the amount of leftover food remaining on a plate. Studies have shown that up to 65% of prepared food is not consumed in health care institutes, which is a higher level than can be observed in other food service settings. These high levels of food waste can lead to economic losses for the entity serving the food. Strategies to minimise food waste include reducing portion sizes, adapting meal compositions with adapted meal item quantities, providing nutritional fortification additives, using bulk meal delivery systems rather than plate services, providing feeding assistance, providing adequate dining environments and facilities, and providing for protected meal times.

Food waste management systems are known in which a plate of left-over food is weighed, the tare weight is subtracted from the weight of the plate with the left-over food and the weight of the left-over food is calculated and stored. The system also comprises a user interface to allow a user to manually input the types of left-overs that are being weighed and a reason for the food becoming waste.

Food logging systems are known in the art, providing various automated approaches for categorising food and estimating their nutrition content. Machine learning techniques have been used along with image-based models of nutritional content of meals. However, in order to ensure reliable operation of such systems once they have been deployed, it is usual practice to refine the models through learning or training sessions performed using real images and involving technical personnel. In a typical patient care environment such technical personnel would usually not be medical staff, and as such, may be referred to as non-medical personnel. Since the real images may contain or be relatable to a patient's private information which must not be divulged to non-medical personnel, the use of such techniques in a patient care or hospital environment presents a problem of protection of personal data.

More generally, automated systems using computer vision technology are known in the hospitality industry for verifying that a particular consumer receives the correct serving of food. Again, wherever such computer vision technology involves machine learning techniques, requiring intervention from non-medical personnel, care has to be taken as to the handling of patients' confidential information, such as personal data, meaning that such systems are not readily useable in a medical facility such as a hospital or a care home.

### BRIEF DESCRIPTION

The present disclosure describes a system and a method for use in a care facility or hospitality environment where consumers, or patients, are subject to a personalised pharmacological regimen or nutritional regimen. By regimen it is meant that pre-determined doses of certain drugs, nutrients, food quantities or caloric requirements or limits have been established for a particular consumer or patient. Such drugs, nutrients or foodstuffs can therefore be referred to as care items or items of care provided to consumers or patients according to a pharmacological or nutritional regimen established for the particular consumer or patient. Items of care may therefore be referred to as ingestible items. As mentioned above, as well as being applicable to care facilities or hospitality environments, embodiments described herein may find use in any environment in which consumers within that environment are subject to a nutritional or pharmacological regimen which needs to be monitored or otherwise controlled. Such environments may include institutions such as schools, prisons, training camps, holiday camps and the like. All such environments are included when reference is made to care facilities or hospitality environments in the description which follows.

In view of the prior art, there is a desire and a need for an economical and comprehensive determination and documentation of the food consumption of individual patients within a health care unit. First of all, there is a need to ensure that the correct meal is delivered to the correct individual. Furthermore, there is a need to quantify the weight or volume and type of consumed food and/or discarded food and to relate this to the consumption requirements and/or preferences of a particular individual. Finally, there is a need to adapt the future preparation of food for the particular individual to optimise the quantity, quality and composition of the food proposed for the individual in terms of his or her nutritional and/or medical requirements and/or his or her personal preferences, thereby providing an opportunity to reduce the amount of discarded food. By nutritional requirements, it is meant the calorific content, carbohydrate content, protein content, vitamin content, mineral content, salt content, fibre content, fats content, and so on, of the food. By medicinal requirements it is meant pharmacological content of the food or of an additive to the food.

Embodiments described herein provide for a qualitative and quantitative analysis of the food provided to patients to be performed in order to ensure that the right patient received the right food and in the right quantities. Other embodiments provide for an analysis of the nutritional consumption of patients to be deduced from the observations of food provided compared to observations of food waste or left-overs. This provides for cases of malnutrition to be detected and for meal preparations to be adjusted accordingly to remedy the nutrition content of the meals. Patient studies may also be undertaken using systems and processes described herein in order to observe relationships between nutrition and patient recovery or nutrition in relation to particular pathologies.

According to a first aspect, provision is made for a computer implemented process for monitoring care provided to a consumer according to a pharmacological or nutritional regimen for the consumer as recorded in a private database, said care including at least one ingestible item being made available on a serving support upon which is also placed at least one indicator comprising: one or more human-readable signs corresponding to the consumer; and a machine-readable visible sign upon which is encoded a consumer code allowing for the consumer to be identified within the private database,
the method comprising:
capturing, using an image capture device, one or more first images of at least the ingestible items and the indicator;
analysing the captured first image using a machine learning model to identify one or more of the ingestible items and to identify human-readable sign on the indicator; and
storing, in a training database, an anonymised version of the captured first image, said anonymised version of the captured first image being generated by electronically obfuscating all or part of the human-readable sign on the indicator identified in the captured first image.

According to a second aspect, there is provided a computer vision system for monitoring care provided to a consumer according to a pharmacological or nutritional regimen for the consumer as recorded in a private database, said care including at least one ingestible item being made available on an identifiable serving support upon which is also placed at least one indicator comprising: one or more human-readable signs corresponding to the consumer; and a machine-readable visible sign upon which is encoded a consumer code allowing for the consumer to be identified within the private database;
the computer vision system being communicably connected to the private database, the private database being accessible by authorised personnel, the system comprising:
   an inspection and analysis unit comprising:
      an image capture device to capture one or more first images of at least the ingestible item and the indicator;
      a processing unit to receive and process the first image from the image capture device and to identify one or more of the ingestible items, the human-readable sign and the machine-readable visible sign; and
      a training database for storing the processed first images;
characterised in that:
   the processing unit is configured to analyse the captured first image using a machine learning model to identify one or more ingestible items and to identify the indicator;
   the processing unit is further configured to anonymise the first images, by electronically obfuscating all or part of the human-readable sign on the indicator identified in the captured first image, before storing them in the training database: and
   the system is configured to be provide a user with access at least to the processing unit and the training base and to deny the user access to the private database.

A machine-readable visible sign is a pattern or image that can be scanned, detected or otherwise read, and interpreted by a computer and which also can be seen by a human. By definition therefore, a machine-readable visible sign will be visible to a human when featured in an image captured by an image capture device. Common examples of machine-readable visible signs include barcodes, QR codes.

### BRIEF DESCRIPTION OF THE DRAWING

Embodiments described herein and any advantages they may provide will be better understood with reference to the enclosed drawing and to the detailed description of the various embodiments, wherein:

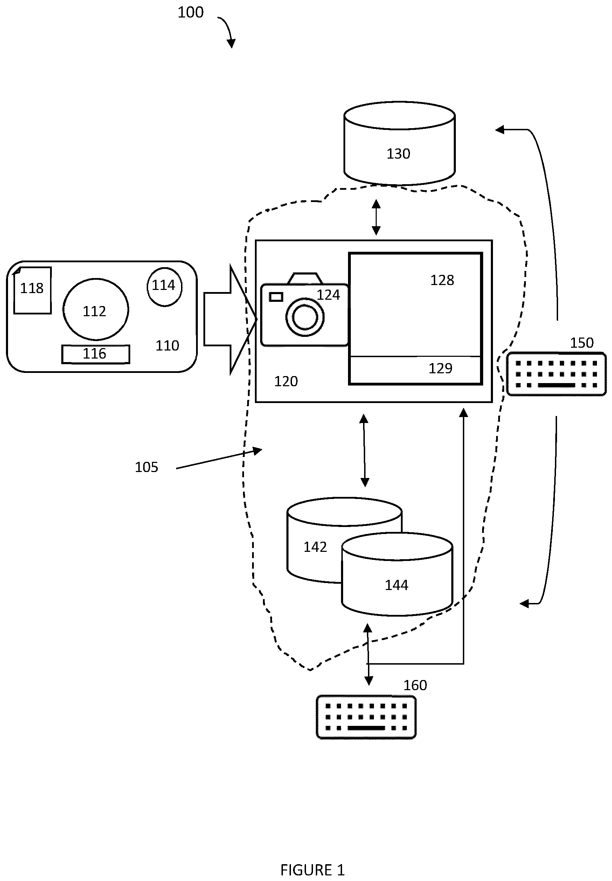

Figure 1 illustrates a system according to an embodiment described herein.

### DETAILED DESCRPTION

Care monitoring in the context of the present disclosure may include verifying that care provided to a particular consumer corresponds to a care regimen established for that consumer, signalling whenever the care provided does not correspond to the regimen established for the consumer and/or documenting the care provided to the consumer. It can be said then, that monitoring care provided to a consumer according to a pharmaceutical regimen means monitoring a type and/or a quantity of a drug or medicament provided to the consumer. Similarly, monitoring care provided to a consumer according to a nutritional regimen means monitoring types and quantities of food or food supplements provided to the consumer and may also include analysing or estimating nutritional content of the food or food supplement.

The provision of adequate nutrition can go a long way to improving the quality of care provided to a patient in a medical facility such as a hospital. Nutrition may therefore form an integral part of patients' medical therapy. Making sure that the right patient receives the right food in terms of quality, quantity and nutritional content is therefore important. The present disclosure relates to an automated system for ensuring that the right patient receives the right food serving in a hospital environment. The automated system involves the use of computer vision technology using image-based machine learning techniques to recognise food items and non-food items. According to some embodiments, the system is configured to identify, quantify and monitor food consumption of different patients. During use, the system may encounter food items and non-food items which it has not learned and which it has to recognise and identify. The system may have access to a menu of images of possible food items and non-food items that it may encounter, thus helping it to decide on what it is seeing. **In** a preferred embodiment, machine learning techniques are employed for recognising and identifying different foodstuffs or food items and non-foodstuffs or non-food items, As with many machine learning systems, once deployed in the field, the ability of the system to recognise different foodstuffs and non-foodstuffs may be improved over time by providing training sets of images representing meals and/or leftovers which the system is likely to encounter during its deployment. The images used for training may also include the non-food items that the system is likely to encounter and therefore capture in one or more images. Such training or other system improvements, requiring access to images captured during the time that the system is deployed, is usually carried out by technical personnel rather than medical personnel. As such, it is important, in order to guarantee the protection of the confidentiality of the consumers' personal data, that personal information related to the patients cannot be accessed by the technical personnel involved in the training or maintenance. Embodiments described herein provide for this patient confidentiality to be maintained while allowing the technical staff to access the image data encountered during the deployment of the system in real life. As well as a patient's identity or contact details, personal information may include, but is not limited to, the patient's food allergies, food-drug interactions, religious dietary requirements, food preferences and communicated desired food quantities. Personal information may further include a patient's recommended nutritional quantities (minimum, maximum calories; minimum, maximum uptakes of particular nutritional components such as proteins, vitamins, etc.).

The step of preparing the food for patients in a health care facility may be achieved through an automated process. In other cases, the step of preparing the food may be a manual process. Embodiments of the secure automated inspection system and computer implemented process for monitoring delivery of meals to a patient described herein may be deployed within a facility where the preparation of the food is done by manual means, with the system ensuring that the correct patient receives the correct food serving. Other embodiments may be deployed within a facility where the preparation of the food is also part of the automated process.

The food is prepared for a particular consumer or patient. Consequently, a health care facility may have a database to store certain data relative to individual patients in their care, such as name, address, age, sex, weight, health status, any particular food allergies, dietary requirements based on medical history, food/drug interactions, drug/drug interactions, other special dietary requirements (such as those based on religious reasons and socio-economic status), particular food preferences and the proposed meal preparations for the up-coming meals, for example. According to an embodiment described herein, this database may be a private database (130) and such data may be used in an automated process for the preparation of a meal for a particular patient. Furthermore, by arranging for the private database to be updated with information related to the food actually provided to each patient, traceability becomes possible. Still further, when information is added to the private database relative to the amount and type of food left over by the patients, the actual food consumption by each patient also becomes traceable. Analysis of the data related to the food provided to each patient and the food left over by each patient allows for the optimisation of the preparation of the food for each patient and thereby leads to a reduction in the quantity of food left over. Hence, through the use of the process and system described herein, the overall quantity of food prepared and discarded by the health care facility may be adjusted, thereby reducing the associated costs.

In general, a private database as described herein may be a database of the care facility in which confidential, medical and/or personal information related to the patient or consumer is stored. The private database may include consumers' food allergies, food-drug interactions, religious dietary requirements, food preferences and any desired food quantities communicated by the consumer. The private database may further include medical information related to the consumers, which may include the consumer's recommended nutritional quantities (minimum/maximum calories; minimum/maximum preferred intake requirements of particular nutritional components such as proteins, vitamins, allergens or foods to be avoided, notes on food/drug interactions or drug/drug interactions, etc.).

According to embodiments described herein, an inspection and analysis unit is provided for automatically recording particular characteristics related to the food being served and for again automatically recording particular characteristics of the remains of the food once the patient has finished eating. According to a particular embodiment, the inspection and analysis unit may comprise a pre-consumption inspection and analysis unit and a post-consumption inspection and analysis unit. Thus, a separate inspection and analysis unit may be provided for recording the characteristics of the food when it is prepared and another separate inspection and analysis unit may be provided for recording the characteristics of the remains of the food. The data thus gathered is analysed, interpreted and fed back into the private database. The inspection and analysis unit, according to an embodiment, comprises one or more sensors of one or more types. One type of sensor may be a weight sensor to weigh the food on a plate to be delivered to the patient. The plate of food may be delivered on a tray or other serving support on which other items may be placed, like a drink for example. The tray may also have non-food items such as cutlery, tableware, packaging items, napkins or a sheet of paper or other human-readable support with the name of the patient for whom the food is intended. According to the embodiment, at least one of the sensors is an image capture device for capturing one or more images of the food, plate and tray and any other items on the tray. The image capture device may be a camera for capturing one or more images or a video camera for capturing a plurality of image frames. Plural cameras may be used as the image capture device, for capturing images from different viewpoints. The image capture device may capture electromagnetic radiation from different parts of the electromagnetic spectrum, for example the image capture device may be one or more infrared cameras.

According to some embodiments, the analysis may be performed for an individual patient. According to other embodiments, the analysis may be performed globally, thus allowing for statistical results to be derived for a particular ward or for the facility in general for example. Similarly, the analysis may allow for studies of food consumption in relation to particular pathologies to be performed, or particular groups of patients, related by age or sex and so on.

Figure 1 illustrates an automated monitoring system (100) according to an embodiment. The system is configured to monitor care provided to a consumer within a care facility according to a pharmacological or nutritional regimen for the consumer as recorded in a private database of the care facility. The care provided may be meals or drugs for example. Since monitoring care provided to a consumer according to a pharmaceutical regimen means monitoring a type and/or a quantity of a drug or medicament provided to the consumer, it follows then, that an item of care would be a medicament or a drug, and since monitoring care provided to a consumer according to a nutritional regimen means monitoring types and quantities of food or food supplements provided to the consumer, which may also include analysing or estimating nutritional content of the food or food supplements, it follows that an item of care in such a case would be a meal component, ingredient or supplement, for example. In all cases then, such items of care can be referred to as ingestible items. By monitoring meals or drugs, or more generally ingestible items, at the time of delivery, the system can ensure that the particular consumer, or patient, gets the meal or medical treatment which was intended for that consumer. The system may be configured to provide a warning, for example an audible or visible warning or a note in a log in a database, whenever the tray intended for a particular consumer has an ingestible item which does not match with the nutritional or pharmacological regimen for that consumer, for example whenever the system detects that the meal provided for the consumer contains an allergen noted for that consumer in the private database. In an embodiment where meals are monitored, by further monitoring the remains of the meal when the patient has finished eating, a record of the patient's consumption may also be kept. The record may be used to adjust the meal content at the next meal delivery time to reduce waste while meeting the nutritional requirements of the patient. An inspection and analysis unit (120) is provided to monitor the delivery of the meal. The same inspection and analysis unit (120), or another inspection and analysis unit, may be used to monitor the remains of the meal when the patient has finished eating. According to a particular embodiment, the inspection and analysis unit includes computer vision technology and is configured to operate using machine learning techniques to analyse image data captured by one or more cameras of the computer vision apparatus.

According to an embodiment, the monitoring system (100) comprises a computer vision system (105) comprising an inspection and analysis unit (120) for inspecting contents of a tray (110) and analysing said contents to try to identify them. This is known as monitoring. Inspection may be done when the tray is delivered to an identified patient, the tray having a serving of food including, for example a glass (114) and a plate (112) with the serving of food, or the meal, comprising one or more food components. The tray (100) may further have cutlery (116) and may still further have other non-foodstuffs, like a napkin, for instance. According to the embodiment, the inspection and analysis unit comprises at least one image capture device (124), such as a camera, for capturing one or more images of the tray and its contents including the meal. Advantageously, the tray may also have a slip of paper with a machine-readable code for identifying the patient for whom the meal on the tray is destined. The slip of paper may also have the patient's name written or printed on it so that a care worker of the facility can readily read the slip and deliver the tray and its contents to the intended patient. The slip of paper serves as an indicator (118). On one hand it indicates to the care worker, via its human-readable inscription, the patient for whom the meal is intended and on the other hand it indicates to a computer vision unit of the system, via a machine-readable code such as the bar code or a QR code or any code which can be read and decoded by a scanner and a decoder, the patient for whom the meal is intended. A QR code or a bar code are both examples of visible signs which can be read and interpreted using computerised means, for example using an image capture device, such as a scanner, and decoded using a decoder. The scanner and the decoder may form a part of the computer vision unit or may be at least partly comprised in a processor of the inspection and analysis unit. In embodiments in which the preparation of the meal is automated, the tray may also be identifiable by having a machine-readable code attached, for example an RFID tag may be attached to the tray so that the tray is identifiable. In embodiments in which a comparison of the tray contents before and after the patient has eaten, the machine-readable code of the tray provides for traceability of the tray through the process. The inspection and analysis unit has access to the private database (130) of the care facility. The private database allows for the code corresponding to the patient, for example the QR code or the bar code present on the indicator, to be linked to the patient's name. The patient's name or identity is considered to be private data. In other words, the private database allows for the identity of the patient to be known and may also allow for other private data related to the patient to be known, for example data concerning a pharmacological regimen or a nutritional regimen of the patient. As will be described further below, the system is able to compare the content of the tray with the intended regimen of the patient and to signal whether the meal is correct or not.

The inspection and analysis unit (120) further comprises a processor or processing unit (128) to receive and analyse or otherwise process the images of the meals captured by the image capture device (124). According to an embodiment, the processing unit (128) includes artificial intelligence hardware and software configured to provide computer vision functionality using machine learning techniques. Machine learning techniques may be realised using neural networks, decision trees or support vector machines for example. In the domain of computer vision, for example, where functions of recognition and identification of objects in captured images are required, it is known to use convolutional neural network technology (CNN) to realise suitable machine learning algorithms for the purpose.

In the domain of machine learning, an engineer or programmer provides a suitable data set of mappings between inputs and their respective desired outputs. The data set is fed into a machine learning algorithm, realised as a neural network for example, and this trains a model to learn a function that produces the mappings with a reasonably high accuracy. With a suitable data set of mappings and with appropriate selection and subsequent tuning of the algorithm by the engineer following evaluation of the model's performance, the model can give reasonably accurate results on a set of test data. When properly tuned, the model can be trained to accurately recognise and identify objects in the images captured by the image capture device.

Embodiments disclosed herein, once deployed within a facility, can be further trained to better recognise the meals and left-overs pertaining to that particular facility by training the machine learning algorithm realised in the processing unit (128) using image data captured by the image capture device during actual use of the system. Consequently, in order to improve the system, technical personnel need to be able to access real image data stored by the system during operation. Paradoxically however, such technical personnel should not have access to private data which would allow a consumer's personal identity to be discovered or for private information related to the patient to be discovered, and such private data could be revealed through access to the real image data should no particular precaution be taken. According to an embodiment, the image data captured during operation may be stored in a training memory or training database (144). The training database is accessible by users such as the technical personnel in charge of updating, teaching or otherwise improving the operation of the computer vision system for recognising and identifying the food items of the meal or the components of the left-overs. Advantageously, in all embodiments, the inspection and analysis unit is configured to anonymise the image data before it is stored in the training database. Furthermore, such technical personnel are prohibited, using electronic means such as electronic enforcement of access control rights, from accessing the private database. Medical staff having the appropriate access rights may access (150) the private database. Access to the private database by users who are not medical staff is thus excluded.

By anonymising it is meant ensuring that any part of the image data stored in the training database does not allow for a patient's identity to be discovered or for a patient's personal or private information to be discovered by unauthorised personnel. Unauthorised personnel includes anyone who is not authorised to access information related to the patient where such information has been deemed to be of a private, personal or otherwise confidential nature. The technical personnel in charge of training or otherwise modifying the machine learning system of the inspection and analysis unit are therefore considered to be non-authorised personnel. In facilities such as hospitals, the patient's doctor could be considered to be authorised personnel, for example. Confidential information may include any information related to the patient's medical treatment, especially when such information can be linked to the patient.

As mentioned above, the tray has an indicator on it, which includes the consumer's name or some way for a worker within the care facility to easily see for which patient the meal is intended. Examples of anonymising include obfuscation, using electronic means, of all or part of the paper on which the patient's name appears. Any electronic obfuscation technique may be used, for example by performing image processing techniques on the image data to blur at least a part of the information on the paper, preferably blurring all or a substantial part of the consumer's name. Other electronic obfuscation techniques include performing image processing techniques to replace a part of the image, preferably a part in which the consumer's name would be visible, by image data which does not reveal the consumer's name, thus effectively obscuring or otherwise hiding all or part of the consumer's name on the paper. Blurring a part of an image or replacing a part of an image with replacement image data may be done using encryption techniques, for example. Any of the above techniques are examples of techniques which may be used to provide electronic obfuscation for anonymising the image data for storing in the training database, as disclosed herein.

Technical personnel do not have access to the private database but do have access to the anonymised images in the training database. The anonymised images are realistic enough to serve the purpose of training the machine learning processor (128) without revealing any private data related to the consumer. Thus, technical personnel in charge of training will not be able to discover any private or personal information since they can only access the anonymised images in the training database, without having access to the private database.

According to embodiments described herein, the inspection and analysis unit is further configured to inspect, observe or otherwise document the remains of the meal after the consumer has finished eating and to evaluate a difference between the meal observed at delivery time and the remains of the meal observed at collection time when the consumer has finished eating.

The private database may store records for a number of different consumers at the health-care facility, comprising an identifier of the patient and details related to the patient, such as age, sex, weight, medical condition, required food intake per meal or per day in terms of energy requirements, nutritional requirements, dietary requirements, for example. Staff for whom authorisation has been cleared may have access to the private database, via a user interface (150), to update certain data relating to individual consumers should his or her medical or care condition evolve, resulting in updated food intake requirements in the private database for that consumer, for example. The private database may also store metrics associated with particular foodstuffs used in the ingredients of meals susceptible to be provided for the consumers. Such metrics may include, for example, calorific (energy) content per serving amount, or nutritional content per serving amount, such as vitamin content or mineral content, or optimum temperature that a particular foodstuff should be served, and so on. The training database may hold reference images of particular foodstuffs from the care facility's menu in order to allow for automated estimation of food types and quantities or volumes. Figure 1 shows a menu database (142) for storing the images from the menu. The menu database may have the same access rights as the training database in that it is accessible (160) to users, such as technical personnel, who have access to the training database. Medical person may have access (150) to the private database, the private database and the training database. The different levels of access may be managed using electronic access control means.

Food may be prepared manually or using automated means at a meal preparation unit. For reasons of freshness and for maintaining the food at the required temperature, the meal preparation unit is preferably located within the health-care facility where the patients are located. **In** other cases, the meal preparation unit may be located at some external facility, especially when adequate provisions are made to maintain the food at the required temperature and to deliver it to the health-care facility quickly. The meal preparation unit has access to the private database. According to one embodiment, the meal preparation unit may be a kitchen comprising cooking equipment for preparing different foodstuffs used in the preparation of meal servings for patients. The meal preparation unit may have a computer work station connected to the private database via a network connection, the workstation being configured to provide instructions, to a cook, for the preparation of personalised meals for each patient based on the patients' records in the private database and on the nutritional content and/or the calorific content and/or the pharmacological content of different ingredients of the meals according to the private database. The instructions may further be based on a stored list of suggested menus. The workstation then provides the amounts of each ingredient of meals on a patient to patient basis. Either a person prepares the serving of the meal following the instructions provided by the workstation or an automated food dispenser uses the instructions to automatically prepare the meal servings. In order to identify which meal should go to which patient, the servings may be delivered to the relevant patient on a tray which has an electronic identification system such an RFID tag. Other types of electronic identification are also possible for the tray, such as a QR code or a bar code, which both use optical scanning techniques.

According to embodiments described herein, an inspection and analysis unit (120) is provided to inspect the prepared meal to be delivered to a particular patient and, according to an embodiment, to further inspect the remains of the meal at collection once the patient has finished eating. Inspecting in the context of the embodiments described herein means recording at least one characteristic of the meal or the remains of the meal. Such a characteristic may be the weight of the meal or of the remains, for example. Another characteristic could be the composition of the meal or of the remains of the meal in terms of the ingredients or in terms of volume. The inspection and analysis unit may also be configured to provide an alert should the meal not correspond to the patient for whom the meal is intended, according to the private database and a tray identifier on the tray on which the meal is placed. The inspection and analysis unit may also be configured to provide an alert should the meal not correspond to the planned meal preparation for the particular consumer, in terms of food item composition (recipe), according to the private database and a tray identifier on the tray on which the meal is placed. The inspection and analysis unit may also be configured to provide an alert should the meal item quantities not correspond to the planned quantities for the consumer, according to the private database and a tray identifier on the tray on which the meal is placed.

According to a variant, separate equipment may be provided to inspect the prepared meal and to inspect the remains of the meal. In such a case, it can be said that the inspection and analysis unit comprises a pre-consumption inspection and analysis unit configured to measure at least one characteristic of the prepared serving of the meal and a separate post-consumption inspection and analysis unit configured to measure at least one characteristic of the remains of the meal when the consumer has ceased consumption of the prepared serving.

A system in which an embodiment of an inspection and analysis unit may be deployed may comprise an RFID reader to read an RFID tag on a tray on which the prepared meal is placed or on which the remains of the meal are placed. The RFID tag may store an identifier of the tray, which may allow for the consumer for whom the tray is intended to be identified. The RFID tag thus allows for traceability of the tray so that the contents of the tray can be compared at the time of delivery and the time after the meal is consumed. The inspection times at delivery and after consumption may also be recorded in the private database or the training database as a time/date stamp corresponding to the respective case. The embodiment may further comprise a weighing scale for weighing the prepared meal and/or the remains of the meal. The scale is preferably an electronic scale or at least a scale whose measured value can be converted to an electronic value so that the system can use the measured value. The pre-consumption inspection and analysis unit may be configured to verify that the correct meal has been prepared to a particular consumer. A check may also be made that the correct ingredients have been used for the meal.

In some embodiments, where it is desirable to estimate the composition of the meal or the remains in terms of their ingredients, the inspection and analysis unit may further comprise one or more cameras for capturing one or more images of the meal or the remains of the meal. A multiple-sensor based food item recognition system, for example one which comprises an inter-related plurality of measurement devices and which is configured to use a combination of the measured weights and the images provided by the sensors and to use this to estimate which ingredients are present can be used to more accurately identify and quantify the contents of the meal or the remains. Multiple cameras may be used, for example, with different cameras being sensitive to different wavelengths of light in order to better identify the ingredients. Infra-red cameras may be used as temperature sensors. Three-dimensional images of the food or the remains may be used to determine the volume of the food or remains. Three-dimensional reconstructions derived from camera systems may be used.

In another embodiment, which may be combined with any of the previous embodiments, the inspection and analysis unit may further comprise a temperature measurement unit to monitor the temperature of the food upon delivery and/or upon collection since this data may be useful in determining a reason for why a patient might be throwing away all or part of the food delivered. Temperature of the food may be used as an indication contributing to the perceived quality of the food.

In another embodiment, which may be combined with any of the previous embodiments, the inspection and analysis unit may further comprise a volumetric determination unit to establish the volume of the food item upon delivery and/or upon collection since this data may be useful in calculating the caloric value of a food item, among others.

In a particular embodiment, provision is made for further input to be given relative to the appreciation of the meal. This may provide a further indication corresponding to the perceived quality of the meal. For example, as shown in figure 1, provision may be made for a user to input an appreciation, for example regarding the volume of the remains or the make-up in terms of ingredients of the remains. The user may be a health-care worker or a person from medical staff or the user may be the patient or consumer. The patient may be allowed to input his or her personal preferences, to be taken into consideration for future meal preparation. Alternatively, or in conjunction, the patient may be allowed to input a reason for having either consumed all of the meal or having left some or all of the meal. According to other variants, the input of such appreciations may be provided at another stage such as the inspection and analysis stages, which will be described later.

An embodiment of a system described herein may further be configured to calculate information relative to the consumer's consumption of the prepared serving based on the measurements from the inspection and analysis unit or the pre-consumption and post-consumption inspection and analysis units. Different actions such as retrieving and adding data to the private database, automatically executing mathematical calculations, interpreting data by a trained individual or automatically to come to recommendations to be added into the private database are tasks executable in a modern IT environment which may include delocalised computing units or alternatively with discrete computing units linked in a local network.

The inspection and analysis unit may temporarily store information pertaining to each consumer's consumption of the prepared meals and/or the further appreciations described above. The inspection and analysis unit may also be configured to process and analyse this information. As mentioned before, the inspection and analysis unit has access to the private database. The inspection and analysis unit may update private database entries for different consumers depending on the results of the analyses performed. For example, if the result of an analysis shows that a particular consumer has finished the whole meal because of an insufficient quantity or has not finished a meal because of a dislike of a certain ingredient or due to the wrong serving temperature of a certain ingredient, then this information may be fed back to the private database in order for the preparation of further meals for that particular patient to be altered accordingly.

Health care facilities generally prepare several meals a day for each patient. Systems and methods according to embodiments described herein allow for medical requirements and the patient's preferences to be taken into account in the preparation of the food for that individual patient and may rely on data such as the patient's age, sex, weight, health status, food allergies, dietary requirements, religious food requirements, food preferences and the like. The requirements and preferences may be documented, preferably in electronic form, in the private database. Furthermore, some health care facilities allow the patients to select a menu or compose their menu individually, within the limited frame of their dietary regimen. The menu selections may be documented, preferably in electronic form in the private database. The meal preparation unit, having access to the private database, can then take this information into account for the preparation of the next upcoming meal for a particular patient. The information may be provided to the preparation unit in hardcopy form or, preferably, in electronic form.

The meals may be prepared and conditioned into individual and identifiable trays at the preparation unit. The tray may be a multi-compartment tray, or there may be one or more plates placed on a serving tray. A meal prepared specifically for an individual patient requires to be identifiable and is associated with, or corresponds to, the particular patient. This may be achieved by a simple piece of paper carrying the patient's name. Preferably however, in order to automate the system, such a paper may carry a QR code or a bar code. Alternatively, any other attachable support other than a paper may be used. For example, an RFID tag may be attached directly onto the tray or may be integrated into the tray. Other means may rely on Bluetooth, Wi-Fi or radio frequency transmission and reception.

During or after the food preparation in the preparation unit, different characteristics or metrics of the prepared food may be recorded and transmitted to the inspection and analysis unit where the characteristics or metrics may be stored along with an identifier of the patient for whom the meal is intended to be delivered. The inspection and analysis unit may comprise one or more types of equipment relying on different technologies to record food metrics or characteristics. For example, one or more photos may be taken by one or more cameras at the inspection and analysis unit after the dish with all food components is prepared. The photos may be transmitted to the inspection and analysis unit and subsequently analysed in order to interpret their meaning. The private database may then be updated depending on the results of the analysis. The photographic representation may be used for purely documentation reasons, providing photographic evidence of the meal and its components prepared for a patient. In an advanced form of analysis of photographic representations, especially from photos taken from more than one camera, quantification of food components can be executed from photographic data, for example by modelling a 3D representation of the food component and hence determining the weight of the corresponding components or ingredients. Instead of relying on 3D representations built from cameras, radar or LiDAR systems, laser scanners or ultrasound scanners can be used to determine the volume of a food item. If thermal cameras are used, cameras recording wavelengths also in the non-visible infrared part of the spectrum, the temperature of the food components can be determined and documented.

Details regarding the required preparation of a particular patient's meal are communicated from the private database to the meal preparation unit, where the relevant menu components may be selected, either by hand or by an automated ingredient selection unit, and placed onto an identifiable tray for the particular patient. As mentioned above, the tray may be rendered identifiable for a particular patient through the use of an RFID tag for example. At the inspection and analysis unit, a digital scale may be used to determine the tare of the tray and any other equipment placed thereon, such as cutlery, and subsequently to determine the quantities of individual food components after each addition of the respective food component to the tray, or the removal of the food component as the case may be. Alternatively, the scale may be used to weigh the total weight of the food ingredients or the total weight of the food, the tray and any other items on the tray, such as cutlery. In some embodiments at least one camera may be used to take a photo of the tray as well. RFID data, all determined weights, photos, time stamps of the corresponding metric levy may be sent to the inspection and analysis unit and stored along with the identifier of the patient. Cutlery and any other non-food items added to the tray may be taken into account in a way which it will not interfere with the weighing and photographing, e.g. by adding such pieces after the weighing and photographing. Alternatively, the captured image or images of the tray may be used to deduce which non-food items were present on the tray and their presence duly taken account of.

As mentioned above, some embodiments may rely on weight information supplied by the inspection and analysis unit. For example, a pre-consumption inspection and analysis unit may be placed at the food preparation unit or an area where the food is prepared. The tray may be weighed without cutlery or glasses etc. The tray may then be weighed with an empty plate for the food in order to obtain the tare weight of the plate. If necessary, the tare weight of tray plus plate plus cutlery may be measured. Following the addition of each component of food to the tray, the weight could be measured again in order to find the weight of the particular component. For example, one component could be potatoes, another component fish and a third component vegetables. According to other embodiments, instead of weight information being used, image information may be used. The image information may further be used to estimate a volume of the food. The volume of the food, given a particular food type, may further be used to calculate the calorific content. According to yet another embodiment, a combination of weight information and image information may be used. Similarly, according to the different embodiments, either weight information, image information or a combination of image and weight information may be used during the post-consumption inspection phase.

Once the meal has been prepared, the prepared meal is then delivered to the patient. In some embodiments, inspection of the prepared meal may be carried out at the meal preparation unit, as described above. In other embodiments, inspection of the prepared meal may be carried out directly at the place where the food is delivered and where the patient will consume it. This has advantages especially when characteristics related to the food inspection include temperature measurements or the time of delivery. An RFID reader may be used at the place where the meal is delivered to the patient to ensure that the patient and the tray delivered to the patient are properly matched. The results of the inspection of the prepared meal may be transmitted to the inspection and analysis unit using any available transmission means such as Bluetooth or Wi-Fi, where the data may be stored along with the identifier of the patient.

Depending on the services available at a health care facility, after the consumption of the meal the patient may be invited to give his or her appreciation of the menu and the different food ingredients. A feedback may cover details regarding the perceived quality and taste as well as sufficiency in terms of the quantity of the meal in general or of certain ingredients or components. The time of delivery of the meal may also be a criterion which is of use in the feedback.

After the patient has finished with his or her meal, a further inspection of the tray may be made. This further inspection may be carried out at the premises from where the meal was sent to be delivered to the patient, in which case the inspection and analysis unit may be the same inspection and analysis unit which inspected the tray following preparation and before consumption. Alternatively, the food tray may be collected and taken to a food discarding and food receptacle cleaning unit of the health care facility. In some cases, this may be a part of the same unit which was used for the preparation of the food, in which case the inspection and analysis unit may again be the same unit which was used for pre-consumption inspection. Otherwise, if the discarding and cleaning unit is a separate unit, then a further inspection and analysis unit may be used to perform a post-consumption inspection of the remnants of the meal following consumption. Any cutlery or other non-food items may either be removed from the tray or automatically taken into consideration as described previously. The RFID information may be read from the tray. At least one digital camera photograph may be taken of the tray, and a digital scale may be used to record or otherwise derive the weight of the tray. A sequenced removal of the food components with intermediate weighing may be executed to recover weight information on the individual remnant food components. RFID data, all determined weights, photos, time stamps of the corresponding metric levy may then be transmitted to the inspection and analysis unit and stored along with the identifier of the patient.

The inspection and analysis unit may be described as a data collection and interpretation system and may be integrated with an IT system and network of the health care facility. It can be a data base and a software located on a server or it can be a dedicated personal computer.

As mentioned above, analysis of the data may be performed at the inspection and analysis unit by the machine learning processor mentioned above (128). In cases where a separate general processor or general processing unit (129) is provided for receiving the data from the machine learning or artificial intelligence processor (128) of the inspection and analysis unit, this general processing unit (129) may perform any necessary analysis of the data and may update the private database or the training database accordingly.

As mentioned above, the artificial intelligence part of the system can be used to calculate or estimate the weight of the food on the tray when it is delivered to the consumer and the leftover food when the consumer has finished eating. In some embodiments, where a general processing unit is present, the general processing unit (129) may be used to determine the weight of the totality of the food served to a particular patient, or the weight of the individual meal ingredients. Simple mathematical calculations may be used while taking into consideration the tare of the tray. For example, following a first weight measurement of the prepared food and tray, a subtraction of the tare of the tray yields the weight of the added food. In the case of further additions to the tray and weighing, the formerly determined weight is subtracted yielding the weight of the added ingredient. In some embodiments of meal preparation units a system for identifying the type of individual food components added to the tray may include a tactile IT user surface, allowing a quick touch action to identify a food type and thereby allowing for the weight and the type of food component to be determined. In other embodiments, data analysis, the mathematical calculations mentioned above and the updating of the private database or training database may be performed by a part of the machine learning processor where a separate general processor is not provided.

The weight determining procedure is reversed at the discarding unit and again relying on simple mathematical calculations. From the total weight with residual food the tare of tray is subtracted to determine the overall quantity of returned, non-eaten food. In the case of successive removal of food ingredients, after a removal of food component and weighing the tray, this weight is subtracted from the formerly determined weight yielding the weight of the removed ingredient. A system may be put in place for identifying the type of individual food components removed from the tray. An example is a tactile IT user surface at the discarding station allowing a quick touch action to identify a food type and link the weight and the type of food component.

The difference between served food weight and returned food weight determines the quantity of food eaten by a patient. The subtraction of served food component weight minus returned food component weight determines the quantity of consumed individual food components. These values may be used to update the private database. Weight to energy (Calorie or Joule) conversion can be performed depending on the calorific value of the menu or the food component and the energy values of food served and consumed may appear as metrics alongside the respective weights and may be used to update the private database.

Depending on different embodiments, the processing unit (128) or the general processing unit (129) may be used to analyse all the gathered data. According to an embodiment, a trained person, such as a medical doctor or a dietitian, may review the food consumption of a patient. Together with the patient's actual and predicted health status development, as determined by the private database entries concerning the patient, the food portions, including the calorific values of next meals for the individual patient may be modified and corresponding orders provided to the meal preparation unit in order to deliver the appropriate amount and quality of food to the patient to satisfy his or her culinary tastes and to deliver the appropriate nutritional content and consequently minimise the quantity of food returned for discarding. The trained person may follow the success of their proposed measures over the course of some meals and successively improve the quality and quantity of food preparation.

The aforementioned revising of a patient's food amount and make-up may also be executed by software algorithms analysing actual consumption and predicting future consumption of food by taking into account the patient's personals and health records as recorded in the private database.

The aforementioned revising of a patient's food amount may also be monitored by software algorithms analysing actual food consumption and pattern deriving from a typical, expected behaviour can be flagged to a trained person, such as a medical doctor or a dietitian for review. In such an approach, tendencies of malnutrition may be spotted early, and the quality of the health care may be improved.

The elaborated recommendations for a patient's upcoming meal preparations are preferably used to update the private database.

Some embodiments described herein rely on a comparison of an observation of what is delivered to a patient with an observation of what is returned by the patient. In some such embodiments, the comparison may use a weight of a tray of food delivered to the patient and a weight of the tray when the patient has finished with the tray of food. In other embodiments the comparison may use an analysis of a captured image of the tray of food which is delivered to the patient and a captured image of the tray once the patient has finished eating. Still other embodiments use a combination of both weights and captured images. In an embodiment described below no weighing is done before the patient receives the food and no images are captured before the patient receives the food. In all other aspects, the features are the same and the inspection and analysis unit only inspects the weight and/or the captured images once the patient has finished eating. The comparison is then made with the expected characteristics of the delivered article and the inspected characteristics of the collected article when the patient has finished eating. For example, for a patient A, the private database provides the instructions for the preparation of the food. It is therefore already known what the weight of the food should be before delivery. This can be compared with the weight observed by the inspection and analysis unit when the patient has finished. Similarly, it is already known what an image of the delivered tray should look like before delivery to the patient. Comparison can be made with one or more images of the remains when the patient has finished. It is known what a volume of each ingredient would be present somewhere on the plate. This can be compared with a volume of food derived by inspection of one or more images of the left-overs.

According to an embodiment, the processing unit may be configured to recognise non-food items. Recognising non-food items can be considered to be easier than recognising food items. By removing the non-food items from consideration by the processor, further processing to recognise and identify the remaining food items is rendered somewhat simpler.

According to different embodiments, training may be supervised, semi-supervised or unsupervised. In embodiments where training is unsupervised, the processor of the inspection and analysis unit may have access to a menu or recipe of possible ingredients. The inspection and analysis unit also has access to the private database, which also helps the processor to be able to recognise which possible food items appear in the image. It is also possible, through training, for the system to recognise food items which do not appear on the menu. The menu may be one of a number of different types of menu, for example a breakfast menu, a lunch menu, a dinner menu, a snack menu. The menu or recipe may be stored in the training database or the menu database.

The inspection and analysis unit is configured to store the anonymised images in the training database for later use for training the system to recognise and identify items on the tray. Preferably, for reasons of more robust training, the training database should include images of trays before the consumer has begun eating as well as images of trays when the consumer has finished eating. Time information relating to the time that an image was captured may also be included in the training database so that the processor can work out the order in which different images of a same tray, according to an identifier of the tray, such as a QR code or RFID code, were captured.

## Claims

1. A computer implemented process for monitoring care provided to a consumer according to a pharmacological or nutritional regimen for the consumer as recorded in a private database (130), said care including at least one ingestible item being made available on a serving support (110) upon which is also placed at least one indicator (118) comprising: one or more human-readable signs corresponding to the consumer; and a machine-readable visible sign upon which is encoded a consumer code allowing for the consumer to be identified within the private database;
the method comprising:
capturing, using an image capture device (124), one or more first images of at least the ingestible items and the indicator;
analysing the captured first image using a machine learning model (128) to identify one or more of the ingestible items and to identify the human-readable sign on the indicator (118); and
storing, in a training database (144), an anonymised version of the captured first image, said anonymised version of the captured first image being generated by electronically obfuscating all or part of the human-readable sign on the indicator (118) identified in the captured first image.

2. The computer implemented process according to claim 1, further comprising:
further analysing the captured first image to identify the machine-readable visible sign on the indicator (118);
extracting the consumer code from the machine-readable visible sign;
identifying the consumer in the private database (130) by matching the extracted consumer code to the consumer;
accessing a menu database (142) to obtain a pharmacological or nutritional content of the identified ingestible items;
comparing, using a processor (129), the pharmacological or nutritional content of the identified ingestible items with the pharmacological or nutritional regimen of the identified consumer; and
providing a warning if the result of the comparison is negative.

3. The computer implemented process according to any of the preceding claims, further comprising updating a record corresponding to the identified consumer in the private database (130) to record the pharmacological or nutritional content of the identified ingestible items.

4. The computer implemented process according to any of the preceding claims, wherein the serving support (110) comprises a machine-readable code to allow for the serving support to be identified using a suitable code reader, the method further comprising:
upon delivery of said care to the consumer:
automatically identifying the serving support (110) by reading a machine-readable code of the serving support;
correlating the identified serving support with the previously extracted consumer code; and
upon collection of said serving support after the consumer has finished:
automatically identifying the serving support by re-reading the machine-readable code of the serving support;
capturing, using an image capture device, one or more further images at least of any remnants of the ingestible items on the serving support;
analysing the captured further image using the machine learning model to identify one or more of the remnants of the ingestible items and to identify the indicator if present;
further updating the record corresponding to the correlated consumer in the private database (130) to record the remnants of the ingestible items; and
storing, in the training database (144), an anonymised version of the captured further image, said anonymised version of the captured further image being obtained by electronically obfuscating all or part of the human-readable sign on the indicator should the indicator have been identified in the captured further image.

5. The computer implemented process according to any of the preceding claims, wherein said electronic obfuscation involves a process using blurring techniques, encryption techniques or image replacement techniques.

6. The computer implemented process according to either of claims 4 or 5, further comprising:
comparing, using a processing unit (129), the first image and the further image to estimate a consumption amount of the ingestible items by the identified consumer.

7. The computer implemented process according to any of the preceding claims, further including:
updating the machine learning model (128) using one or more first images and/or further images from the training database (144) by a user to whom access to the private database is electronically excluded.

8. A computer vision system (105) for monitoring care provided to a consumer according to a pharmacological or nutritional regimen for the consumer as recorded in a private database (130), said care including at least one ingestible item being made available on an identifiable serving support (110) upon which is also placed at least one indicator (118) comprising: one or more human-readable signs corresponding to the consumer; and a machine-readable visible sign upon which is encoded a consumer code allowing for the consumer to be identified within the private database (130);
the computer vision system (105) being communicably connected to the private database (130), the private database being accessible by authorised personnel, the system comprising:
an inspection and analysis unit (120) comprising:
an image capture device to capture one or more first images of at least the ingestible item and the indicator;
a processing unit (129) to receive and process the first image from the image capture device and to identify one or more of the ingestible items, the human-readable sign and the machine-readable visible sign; and
a training database (144) for storing the processed first images;
**characterised in that**:
the processing unit is configured to analyse the captured first image using a machine learning model (128) to identify one or more ingestible items and to identify the indicator (118);
the processing unit (129) is further configured to anonymise the first images, by electronically obfuscating all or part of the human-readable sign on the indicator (118) identified in the captured first image, before storing them in the training database (144); and
the system is configured to be provide a user with access at least to the processing unit (129) and the training database (144) and to deny the user access to the private database (130).

## Patentansprüche

1. Ein computerimplementierter Prozess zur Überwachung der einem Verbraucher gemäß einem in einer privaten Datenbank (130) aufgezeichneten pharmakologischen oder ernährungsbezogenen Regime bereitgestellten Pflege, wobei diese Pflege mindestens einen auf einer Servierunterlage (110) bereitgestellten einnehmbaren Artikel umfasst und wobei auf dieser Servierunterlage außerdem mindestens ein Indikator (118) angebracht ist umfassend: ein oder mehrere dem Verbraucher zugeordnete, für Menschen lesbare Zeichen; und ein maschinenlesbares sichtbares Zeichen auf dem ein Verbrauchercode kodiert ist, der die Identifizierung des Verbrauchers in der privaten Datenbank ermöglicht;
wobei das Verfahren umfasst:
Aufnehmen mittels eines Bildaufnahmegeräts (124) von einem oder mehreren ersten Bildern, auf denen zumindest die einnehmbaren Artikel und der Indikator abgebildet sind;
Analysieren des erfassten ersten Bildes mittels eines Machine-Learning-Modells (128), um ein oder mehrere der einnehmbaren Artikel sowie das für Menschen lesbare Zeichen auf dem Indikator (118) zu identifizieren; und
Speichern in einer Trainingsdatenbank (144) einer anonymisierten Version des erfassten ersten Bildes, wobei diese anonymisierte Version des erfassten ersten Bildes durch elektronisches Unkenntlichmachen des gesamten oder eines Teils des in dem erfassten ersten Bild identifizierten für Menschen lesbaren Zeichens auf dem Indikator (118) erzeugt wird.

2. Der computerimplementierte Prozess nach Anspruch 1, ferner umfassend:
Weiteres Analysieren des erfassten ersten Bildes, um das maschinenlesbare sichtbare Zeichen auf dem Indikator (118) zu identifizieren;
Extrahieren des Verbrauchercodes aus dem maschinenlesbaren sichtbaren Zeichen;
Identifizieren des Verbrauchers in der privaten Datenbank (130) durch Abgleichen des extrahierten Verbrauchercodes mit dem Verbraucher;
Abrufen aus einer Menüdatenbank (142), um den pharmakologischen oder ernährungsbezogenen Inhalt der identifizierten einnehmbaren Artikel zu erhalten;
Vergleichen, unter Verwendung einer Verarbeitungseinheit (129), des pharmakologischen oder ernährungsbezogenen Gehalts der identifizierten einnehmbaren Artikel mit dem pharmakologischen oder ernährungsbezogenen Regime des identifizierten Verbrauchers; und
Ausgeben einer Warnung, wenn das Ergebnis des Vergleichs negativ ist.

3. Der computerimplementierte Prozess nach einem der vorstehenden Ansprüche, ferner umfassend das Aktualisieren eines Datensatzes, der dem identifizierten Verbraucher in der privaten Datenbank (130) entspricht, um den pharmakologischen oder ernährungsbezogenen Gehalt der identifizierten einnehmbaren Artikel zu erfassen.

4. Der computerimplementierte Prozess nach einem der vorstehenden Ansprüche, wobei die Servierunterlage (110) einen maschinenlesbaren Code aufweist, der es ermöglicht, die Servierunterlage mittels eines geeigneten Codelesers zu identifizieren, wobei das Verfahren ferner umfasst:
bei Lieferung der genannten Pflege an den Verbraucher:
automatisches Identifizieren der Servierunterlage (110) durch Auslesen des maschinenlesbaren Codes der Servierunterlage;
Korrelation der identifizierten Servierunterlage mit dem zuvor extrahierten Verbrauchercode; und
bei Einsammlung der genannten Servierunterlage, nachdem der Verbraucher fertig ist:
automatisches Identifizieren der Servierunterlage durch erneutes Auslesen des maschinenlesbaren Codes der Servierunterlage;
Aufnehmen, mittels eines Bildaufnahmegeräts, eines oder mehrerer weiterer Bilder zumindest von etwaigen Rückständen der einnehmbaren Artikel auf der Servierunterlage;
Analyse des erfassten weiteren Bildes mittels des Machine-Learning-Modells (128), um ein oder mehrere der Überreste der einnehmbaren Artikel zu identifizieren und, falls vorhanden, den Indikator (118) zu identifizieren;
Weiteres Aktualisieren des Datensatzes, der dem korrelierten Verbraucher in der privaten Datenbank (130) zugeordnet ist, um die Überreste der einnehmbaren Artikel aufzuzeichnen; und
Speicherung einer anonymisierten Version des erfassten weiteren Bildes in der Trainingsdatenbank (144), wobei diese anonymisierte Version des erfassten weiteren Bildes dadurch erhalten wird, dass alle oder Teile des für Menschen lesbaren Zeichens auf dem Indikator (118) elektronisch unkenntlich gemacht werden, sofern der Indikator (118) im erfassten weiteren Bild identifiziert worden ist.

5. Der computerimplementierte Prozess nach einem der vorangehenden Ansprüche, wobei besagte elektronische Unkenntlichmachung einen Prozess umfasst, der Verwischungstechniken, Verschlüsselungstechniken oder Techniken zum Bildaustausch verwendet.

6. Der computerimplementierte Prozess nach einem der Ansprüche 4 oder 5, wobei er ferner Folgendes umfasst:
Vergleich der ersten Aufnahme und der weiteren Aufnahme mittels einer Verarbeitungseinheit (129), um die konsumierte Menge der einnehmbaren Artikel durch den identifizierten Verbraucher abzuschätzen.

7. Der computerimplementierte Prozess nach einem der vorhergehenden Ansprüche, wobei er ferner Folgendes umfasst:
Aktualisierung des Machine-Learning-Modells (128) mittels eines oder mehrerer erster Bilder und/oder weiterer Bilder aus der Trainingsdatenbank (144) durch einen Benutzer, dem der Zugriff auf die private Datenbank elektronisch verwehrt ist.

8. Ein Computer-Vision-System (105) zur Überwachung der an einen Verbraucher erbrachten Pflege gemäß einem pharmakologischen oder ernährungsbezogenen Regime für den Verbraucher, wie es in einer privaten Datenbank (130) aufgezeichnet ist, wobei diese Pflege mindestens einen auf einer identifizierbaren Servierunterlage (110) bereitgestellten einnehmbaren Artikel umfasst, auf der außerdem mindestens ein Indikator (118) angebracht ist, der Folgendes aufweist: ein oder mehrere für Menschen lesbare Zeichen, die dem Verbraucher entsprechen; und ein maschinenlesbares sichtbares Zeichen, auf dem ein Verbrauchercode kodiert ist, der die Identifizierung des Verbrauchers innerhalb der privaten Datenbank (130) ermöglicht;
wobei das Computer-Vision-System (105) mit der privaten Datenbank (130) kommunizierbar verbunden ist, die private Datenbank von autorisiertem Personal zugänglich ist, und das System Folgendes umfasst:
eine Inspektions- und Analyseeinheit (120), umfassend:
ein Bildaufnahmegerät zum Erfassen eines oder mehrerer erster Bilder zumindest des einnehmbaren Artikels und des Indikators;
eine Verarbeitungseinheit (129), die das erste Bild vom Bildaufnahmegerät empfängt und verarbeitet und ein oder mehrere der einnehmbaren Artikel sowie das für Menschen lesbare Zeichen und das maschinenlesbare sichtbare Zeichen identifiziert; und
eine Trainingsdatenbank (144) zur Speicherung der verarbeiteten ersten Bilder;
**dadurch gekennzeichnet, dass**:
die Verarbeitungseinheit konfiguriert ist, das erfasste erste Bild mithilfe eines Machine-Learning-Modells (128) zu analysieren, um ein oder mehrere einnehmbare Artikel sowie den Indikator (118) zu identifizieren;
die Verarbeitungseinheit (129) ferner konfiguriert ist, das erste Bild zu anonymisieren, indem sie das gesamte oder einen Teil des für Menschen lesbaren Zeichens am im erfassten ersten Bild identifizierten Indikator (118) elektronisch unkenntlich macht, bevor sie dieses in der Trainingsdatenbank (144) speichert; und
das System konfiguriert ist, einem Benutzer zumindest Zugriff auf die Verarbeitungseinheit (129) und die Trainingsdatenbank (144) zu gewähren und dem Benutzer den Zugriff auf die private Datenbank (130) zu verweigern.

## Revendications

1. Un procédé mis en œuvre par ordinateur pour la surveillance des soins fournis à un consommateur, selon un régime pharmacologique ou nutritionnel le concernant tel qu'enregistré dans une base de données privée (130), ces soins comprenant au moins un élément ingestible rendu disponible sur un support de service (110) sur lequel est également placé au moins un indicateur (118) comprenant : un ou plusieurs signes lisibles par l'homme et correspondant au consommateur ; et un signe visible lisible par machine sur lequel est encodé un code du consommateur permettant d'identifier le consommateur dans la base de données privée;
le procédé comprenant :
capturer, à l'aide d'un dispositif de capture d'images (124), une ou plusieurs premières images d'au moins les éléments ingestibles et de l'indicateur ;
analyser la première image capturée à l'aide d'un modèle d'apprentissage automatique (128) afin d'identifier un ou plusieurs des éléments ingestibles et d'identifier le signe lisible par l'homme sur l'indicateur (118) ; et
stocker, dans une base de données d'entraînement (144), une version anonymisée de la première image capturée, ladite version anonymisée de la première image capturée étant générée en obfusquant électroniquement tout ou partie du signe lisible par l'homme sur l'indicateur (118) identifié dans la première image capturée.

2. Le procédé mis en œuvre par ordinateur selon la revendication 1, comprenant en outre :
analyser davantage la première image capturée pour identifier le signe visible lisible par machine sur l'indicateur (118) ;
extraire le code du consommateur du signe visible lisible par machine ;
identifier le consommateur dans la base de données privée (130) en faisant correspondre le code du consommateur extrait au consommateur ;
accéder à une base de données de menus (142) pour obtenir un contenu pharmacologique ou nutritionnel des éléments ingestibles identifiés ;
comparer, à l'aide d'une unité de traitement (129), le contenu pharmacologique ou nutritionnel des éléments ingestibles identifiés avec le régime pharmacologique ou nutritionnel du consommateur identifié ; et
fournir un avertissement si le résultat de la comparaison est négatif.

3. Le procédé mis en œuvre par ordinateur selon l'une quelconque des revendications précédentes, comprenant en outre la mise à jour d'un enregistrement correspondant au consommateur identifié dans la base de données privée (130) afin d'enregistrer le contenu pharmacologique ou nutritionnel des éléments ingestibles identifiés.

4. Le procédé mis en œuvre par ordinateur selon l'une quelconque des revendications précédentes, dans lequel le support de service (110) comprend un code lisible par machine permettant d'identifier le support de service à l'aide d'un lecteur de codes approprié, le procédé comprenant en outre :
lors de la fourniture dudit soin au consommateur :
identifier automatiquement le support de service (110) en lisant le code lisible par machine du support de service ;
corréler le support de service identifié avec le code du consommateur précédemment extrait ; et
lors de la collecte dudit support de service après que le consommateur a terminé :
identifier automatiquement le support de service en relisant le code lisible par machine du support de service ;
capturer, à l'aide d'un dispositif de capture d'images, une ou plusieurs images supplémentaires, au moins des éventuels restes des éléments ingestibles sur le support de service ;
analyser l'image ultérieure capturée à l'aide du modèle d'apprentissage automatique afin d'identifier un ou plusieurs restes des éléments ingestibles et d'identifier l'indicateur, si présent ;
mettre à jour en outre l'enregistrement correspondant au consommateur corrélé dans la base de données privée (130) pour enregistrer les restes des éléments ingestibles ; et
stocker, dans la base de données d'entraînement (144), une version anonymisée de l'image ultérieure capturée, ladite version anonymisée de l'image ultérieure capturée étant obtenue en obfusquant électroniquement tout ou partie du signe lisible par l'homme figurant sur l'indicateur si l'indicateur a été identifié dans l'image ultérieure capturée.

5. Le procédé mis en œuvre par ordinateur selon l'une quelconque des revendications précédentes, dans lequel ladite obfuscation électronique implique un procédé utilisant des techniques de floutage, des techniques de chiffrement ou des techniques de remplacement d'image.

6. Le procédé mis en œuvre par ordinateur selon l'une des revendications 4 ou 5, comprenant en outre :
comparer, en utilisant une unité de traitement (129), la première image et l'image ultérieure afin d'estimer la quantité consommée des éléments ingestibles par le consommateur identifié.

7. Le procédé mis en œuvre par ordinateur selon l'une quelconque des revendications précédentes, comprenant en outre :
mettre à jour le modèle d'apprentissage automatique (128) par un utilisateur auquel l'accès à la base de données privée est exclu électroniquement, en utilisant une ou plusieurs premières images et/ou images ultérieures provenant de la base de données d'entraînement (144).

8. Un système de vision par ordinateur (105) pour surveiller les soins fournis à un consommateur conformément à un régime pharmacologique ou nutritionnel pour le consommateur tel qu'enregistré dans une base de données privée (130), lesdits soins comprenant au moins un élément ingestible mis à disposition sur un support de service identifiable (110) sur lequel est également placé au moins un indicateur (118) comprenant : un ou plusieurs signes lisibles par l'homme correspondant au consommateur ; et un signe visible lisible par machine sur lequel est codé un code du consommateur permettant d'identifier le consommateur dans la base de données privée (130) ;
le système de vision par ordinateur (105) étant connecté de manière communicable à la base de données privée (130), la base de données privée étant accessible par le personnel autorisé, le système comprenant :
une unité d'inspection et d'analyse (120) comprenant :
un dispositif de capture d'images pour capturer une ou plusieurs premières images d'au moins l'élément ingestible et l'indicateur ;
une unité de traitement (129) pour recevoir et traiter la première image provenant du dispositif de capture d'images et pour identifier un ou plusieurs des éléments ingestibles, le signe lisible par l'homme et le signe visible lisible par machine ; et
une base de données d'entraînement (144) pour stocker les premières images traitées ;
**caractérisée en ce que** :
l'unité de traitement est configurée pour analyser la première image capturée en utilisant un modèle d'apprentissage automatique (128) afin d'identifier un ou plusieurs éléments ingestibles et d'identifier l'indicateur (118) ;
l'unité de traitement (129) est en outre configurée pour anonymiser la première image, en obfusquant électroniquement tout ou partie du signe lisible par l'homme sur l'indicateur (118) identifié dans la première image capturée, avant de la stocker dans la base de données d'entraînement (144) ; et
le système est configuré pour fournir à un utilisateur, au moins, l'accès à l'unité de traitement (129) et à la base de données d'entraînement (144) et pour refuser à l'utilisateur l'accès à la base de données privée (130).
